# FASCICULE DE BREVET EUROPEEN

(11) **EP 2 376 496 B1**
(45) Date de publication et mention de la délivrance du brevet: **12.03.2014**
(21) Numéro de dépôt: 09803824.3
(22) Date de dépôt: 08.12.2009
(51) Int. Cl.: C07D 489/02, C07H 17/04, C07H 17/00, C07H 15/24, A61K 31/70, A61K 31/485, A61P 29/00, A61P 25/04

(54) **SYNTHESE DE MORPHINE-6-GLUCURONIDE OU DE L'UN DE SES DERIVES**
VERFAHREN ZUR HERSTELLUNG VON MORPHIN-6-GLUCURONIDEN ODER VON EINEM SEINER DERIVATE
SYNTHESIS OF MORPHINE-6-GLUCURONIDE OR OF A DERIVATIVE THEREOF

(30) Priorité: 10.12.2008 FR 0806948
(43) Date de publication de la demande: 19.10.2011
(73) Titulaire: SANOFI, 75008 Paris (FR)
(72) Inventeur: DLUBALA, Alain, F-75013 Paris (FR); RIPOCHE, Isabelle, F-63174 Aubiere Cedex (FR); TRECANT, Claire, F-67000 Strasbourg (FR)
(74) Mandataire: Veinante, Aude
(86) Numéro de dépôt international: PCT/FR2009/052445
(87) Numéro de publication internationale: WO 2010/067007

(56) Documents cités:
- WO-A-93/03051
- WO-A-99/64430
- WO-A-2005/063263
- US-A- 3 766 188
- HIDETOSHI YOSHIMURA ET AL: "METABOLISM OF DRUGS. LX.1). THE SYNTHESIS OF CODEINE AND MORPHINE GLUCURONIDES2)", CHEMICAL AND PHARMACEUTICAL BULLETIN, PHARMACEUTICAL SOCIETY OF JAPAN, TOKYO, JP, vol. 16, no. 11, 1 janvier 1968 (1968-01-01), pages 2114-2119, XP000614807, ISSN: 0009-2363 cité dans la demande
- BERRANG B ET AL: "Synthesis of morphine-3,6-di-[beta]-D-glururonide", SYNTHESIS 199710 DE, no. 10, octobre 1997 (1997-10), pages 1165-1168, XP002544615, ISSN: 0039-7881
- FRENSCH K ET AL: "OLIGO ETHYLENE GLYCOL ETHERS OF MORPHINE", LIEBIGS ANNALEN DER CHEMIE, no. 12, 1979, pages 2118-2120, XP002544616, ISSN: 0170-2041
- AUTERHOFF H ET AL: "THE MARQUIS REACTION OF MORPHINE", ARCHIV DER PHARMAZIE (WEINHEIM), vol. 306, no. 11, 1973, pages 866-872, XP002544617, ISSN: 0365-6233

## Description

### DOMAINE DE L'INVENTION

La présente invention concerne un procédé de préparation de morphine-6-glucuronide (M6G) ou de l'un de ses dérivés.

### DESCRIPTION DU CONTEXTE DE L'INVENTION

La morphine est actuellement l'analgésique le plus utilisé dans le traitement des douleurs de moyenne et grande intensité. Cet opioïde est utilisé dans environ 80% des cas de douleur aiguë post-opératoire. Malgré une grande efficacité, l'utilisation de la morphine s'accompagne de nombreux effets secondaires indésirables, caractéristiques des opioïdes, tels la dépression respiratoire, les nausées, les vomissements, l'inhibition du transit intestinal, la dépendance et la tolérance (Minoru Nariata et coll. Pharmacol. Et Ther. 2001, 89, 1-15).

On sait que la morphine subit un important métabolisme qui conduit notamment à la formation de morphine-6-glucuronide (M6G). Ce métabolite pénètre faiblement dans le cerveau en raison de son caractère hydrophile. Il présente une activité analgésique plus puissante que celle présentée par la morphine par administration centrale avec une diminution de la dépression respiratoire, des nausées et des vomissements (Paul et coll. J. Pharmacol. Exp. The 1989, 251, 477-483 ; Frances et al J. Pharmacol. Exp. The 1992, 262, 25-31). La demande de brevet WO95/05831 décrit l'utilisation du M6G sous forme orale pour le traitement de la douleur.

Le M6G a été synthétisé en 1968 par Yoshimurai et coll. (Yoshimura, H.; Oguri, K.; Tsukamoto, H. Chem. Pharm. Bull. 1968, 16, 2114-2119). A échelle industrielle, ce procédé qui repose sur le principe de Koenigs Knorr présente un rendement faible. Il a été modifié en 1997 par Berrang et coll. (Synthesis 1997, 1165-1168). Les métaux lourds présents à l'état de trace dans le produit final sont difficiles à éliminer. Enfin, les sels d'argent doivent être recyclés. Il a par ailleurs été proposé dans la demande de brevet WO 99/64430 un procédé de synthèse de M6G par le biais de la synthèse d'un donneur de glycosyle sous forme d'orthoester en présence de perchlorate de lutidinium. Cependant cette méthode ne présente qu'un faible rendement de glycosylation, de l'ordre de 30%. De plus, ces méthodes qui impliquent une glycosylation en milieu hétérogène avec agitation sont difficiles à mettre en oeuvre à l'échelle industrielle.

D'autres modes de réalisation de la O-glycosylation ont été envisagés, notamment par le biais d'une activation sous la forme d'un imidate (WO 93/03051) ou d'un thioaryle. Ces modes de réalisation qui requièrent une mise en oeuvre dans des conditions particulières à savoir d'anhydricité stricte, c'est-à-dire une teneur en eau inférieure à 100 ppm, et de basse température imposent des contraintes importantes au niveau industriel. De plus, l'activation par l'intermédiaire d'un thioaryle utilise généralement du thiophénol qui dégage une odeur nauséabonde problématique dans le cadre d'une mise en oeuvre à l'échelle industrielle.

Il demeure donc un besoin de pouvoir disposer d'un procédé permettant de produire à l'échelle industrielle de tels dérivés, non seulement avec un rendement plus élevé mais également avec un minimum de contraintes techniques.

Le but de la présente invention est de proposer un procédé de préparation de M6G ou l'un de ses dérivés présentant un rendement d'au moins 60%, dont l'étape de glycosylation est effectuée en milieu homogène, est tolérante à l'humidité c'est-à-dire supportant une teneur en eau allant jusqu'à 3000 ppm et pouvant être réalisée à température de l'ordre de 20°C.

Ce but est atteint par le procédé selon l'invention, lequel comprend l'utilisation combinée d'un dérivé glycosylé avec une activation trihalogénoacétamidate comme donneur de dérivés glycosylés et de dérivés dimorphiniques comme accepteur de dérivés glycosylés, permettant d'obtenir une très bonne stéréo-sélectivité.

On a maintenant trouvé un procédé permettant un rendement satisfaisant avec un minimum de contraintes industrielles.

Aussi selon un premier aspect, la présente invention vise un procédé de préparation de M6G ou l'un de ses dérivés comprenant les étapes consistant :
(i) à faire réagir un composé répondant à la formule (I) suivante : 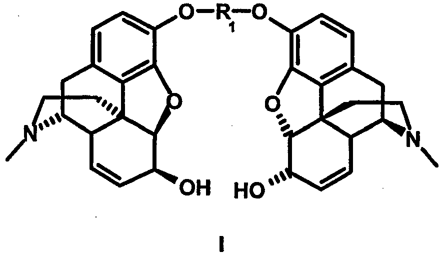 dans laquelle :
   R₁ représente un groupement carbonyle, COR₅CO où R₅ représente un groupe (C₁-C₄)alcane-diyle, (C₂-C₄)alcène-diyle, (C₂-C₄)alcyne-diyle, hétéro(C₁-C₄)alcane-diyle, hétérocyclo(C₃-C₆)alcane-diyle, (C₅-C₁₄)arène-diyle, hétéro(C₄-C₁₀)arène-diyle, bi(C₁₀-C₁₆)arène-oxyde-diyle, ou bi(C₁₀-C₁₆)arène-diyle, SO₂R₆SO₂ où R₆ représente un groupe (C₁-C₄)alcane-diyle, (C₂-C₄)alcène-diyle, (C₂-C₄)alcyne-diyle, hétéro(C₁-C₄)alcane-diyle, hétérocyclo(C₃-C₆)alcane-diyle, (C₅-C₁₄)arène-diyle, hétéro(C₄-C₁₀)arène-diyle, bi(C₁₀-C₁₆)arène-oxyde-diyle ou bi(C₁₀-C₁₆)arène-diyle, avec un dérivé d'acide glucuronique répondant à la formule (II) suivante : 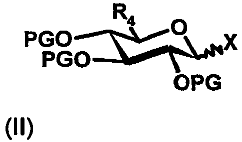 dans laquelle :
      PG représente un groupe acétyle, isobutyryle, benzoyle ou pivaloyle,
      X représente un groupe trihalogénoacétamidate, et
      R₄ représente un groupe (C₁-C₄)alkylcarboxylate,
      en présence :
         - d'un solvant aromatique non substitué ou substitué par un ou plusieurs substituants choisis dans le groupe constitué par un atome d'halogène, un groupe (C₁-C₄)alkyle et un groupe (C₁-C₄)alkyloxy, ledit solvant présentant un point de fusion inférieur ou égal à - 20°C, et
         - de trifluorométhanesulfonyle de triméthylsilane
(ii) à faire réagir le produit obtenu à l'étape (i) avec un agent basique fort, puis
(iii) à récupérer le produit obtenu à l'étape (ii).

La présente invention a également pour objet les composés répondant à la formule (III) suivante 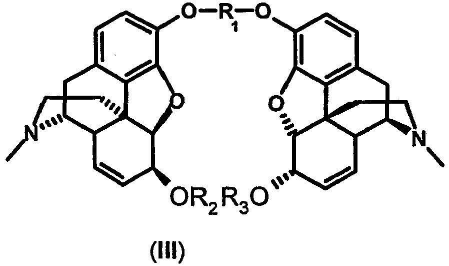 dans laquelle :
R₁ est tel que défini précédemment,
R₂ et R₃ représentent indépendamment un groupe PG tel que défini précédemment ou un groupe répondant à la formule (IV) suivante : 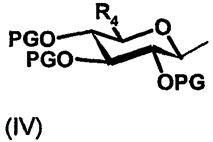 dans laquelle :
R₄ et PG sont tels que définis précédemment,
sous réserve que l'un au moins de R₂ et R₃ représente un groupe de formule (IV).

La présente invention a encore pour objet les composés répondant à la formule (I) suivante : 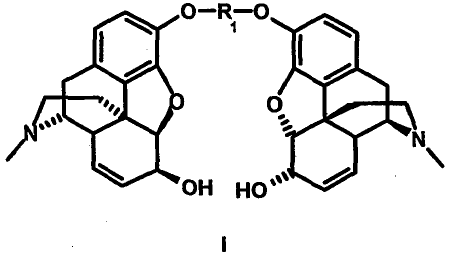 dans laquelle :
R₁ est tel que défini précédemment.

### DEFINITIONS

Dans le cadre de la présente invention, on entend par :
- un groupe PG : un groupe protecteur qui permet, d'une part, de protéger une fonction réactive telle qu'un hydroxy ou une amine pendant une synthèse et, d'autre part, de régénérer la fonction réactive intacte en fin de synthèse ; des exemples de groupes protecteurs ainsi que des méthodes de protection et de déprotection sont données dans « Protective Groups in Organic Synthesis », Green et al., 2nd Edition (John Wiley & Sons, Inc., New York), 1991 ; on citera en particulier les groupes acétyle, isobutyryle, benzoyle et pivaloyle ;
- un atome d'halogène: un atome de fluor, un atome de chlore, un atome de brome ou un atome d'iode ;
- un groupe (C₁-C₄)alkyle : un groupe aliphatique saturé linéaire ou ramifié substitué ou non substitué, ayant de 1 à 4 atomes de carbone ; à titre d'exemples, on peut citer les groupes méthyle, éthyle, propyle, isopropyle, butyle, isobutyle, tertbutyle, etc;
- un groupe hydroxyle : un groupe -OH ;
- un groupe (C₁-C₄)alkyloxy : un groupe -O-(C₁-C₄)alkyle où le groupe (C₁-C₄)alkyle est tel que précédemment défini ;
- un groupe carbonyle, un groupe C=O ;
- un groupe (C₁-C₄)alcane-diyle, un groupe aliphatique saturé divalent linéaire, ramifié ou cyclique, substitué ou non substitué, ayant de 1 à 4 atomes de carbone ; à titre d'exemples, on peut citer les groupes méthane-diyle (-CH₂-), éthane-diyle (-CH₂-CH₂-), propane2,3-diyle (-CH(CH₃)CH₂-), propane1,3-diyle (-CH₂-CH₂-CH₂-) ; etc
- un groupe (C₂-C₄)alcène-diyle, un groupe aliphatique divalent mono- ou poly-insaturé, linéaire ou ramifié, ayant de 2 à 4 atomes de carbone, comprenant par exemple une ou deux insaturations éthyléniques; à titre d'exemples, on peut citer les groupes éthèn-diyle (-CH=CH-), 1-propène 1,3-diyle (-CH₂-CH=CH-), etc.
- un groupe (C₂-C₄)alcyne-diyle, un groupe aliphatique divalent mono- ou poly-insaturé, linéaire ou ramifié, ayant de 2 à 4 atomes de carbone, comprenant par exemple une ou deux insaturations acétyléniques ; à titre d'exemples, on peut citer les groupes éthyne-diyle (-C≡C-), 1-propyne 1,3-diyle (-C≡C-CH2-) ;
- un groupe (C₅-C₁₄)arène-diyle, un groupe aromatique cyclique divalent substitué ou non ayant de préférence entre 5 et 14 atomes de carbone ;
   à titre d'exemples, on peut citer les groupes 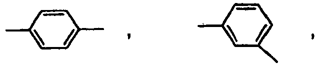 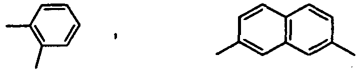
- un groupe hétéro(C₁-C₄)alcane-diyle, un groupe alcane-diyle, tel que défini ci-dessus, substitué ou non substitué, ayant de préférence entre 1 et 4 atomes de carbone, comprenant un ou plusieurs hétéroatomes, tels que l'azote, l'oxygène ou le soufre ; à titre d'exemples, on peut citer le groupe etheroxyde-diyle ;
- un groupe hétérocyclo(C₃-C₆)alcane-diyle, un groupe cyclo(C₃-C₆)alcane-diyle, tel que défini ci-dessus, substitué ou non substitué, ayant de préférence entre 3 et 6 atomes de carbone, comprenant un ou plusieurs hétéroatomes, tels que l'azote, l'oxygène ou le soufre ; à titre d'exemples, on peut citer les groupes oxiranne-diyle, aziridine-diyle, thirane-diyle et pyrane-diyle ;
- un groupe hétérO(C₄-C₁₀)arène-diyle, un groupe aromatique cyclique divalent ayant de préférence entre 4 et 10 atomes de carbone et comprenant un ou plusieurs hétéroatomes, tels que l'azote, l'oxygène ou le soufre ; à titre d'exemples, on peut citer les groupes 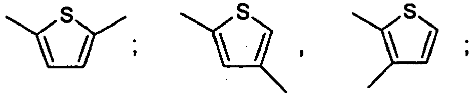
- un groupe bi (C₁₀-C₁₆)arène-diyle, un groupe divalent comprenant deux cycles aromatiques chacun pouvant être indépendamment substitué ou non, ayant de préférence de 10 à 16 atomes de carbone d'exemples, on peut citer le groupe 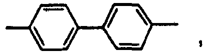
- un groupe bi(C₁₀-C₁₆)arèn-oxyde-diyle, un groupe divalent comprenant deux cycles aromatiques, chacun indépendamment substitué ou non, ayant de 10 à 16 atomes de carbone, à titre d'exemples, on peut citer le groupe 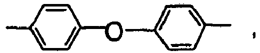 et
- un groupe -(C₁-C₄)alkylcarboxylate, un groupe -CO-O-(C₁-C₄)alkyle, le groupe (C₁-C₄)alkyle étant tel que défini ci-dessus.

L'expression « agent basique fort », désigne de façon bien connue de l'homme du métier tout agent basique qui se dissocie de façon complète en solution aqueuse neutre ou ayant au moins un degré de dissociation élevé en solution aqueuse neutre. L'expression « agent basique fort » désigne en particulier la soude, la potasse, l'hydroxyde de lithium et l'hydroxyde d'ammonium.
- Par l'expression « température ambiante », on entend une température allant de 20 à 25°C.

### DESCRIPTION DETAILLEE DE L'INVENTION

### Composé de formule (I)

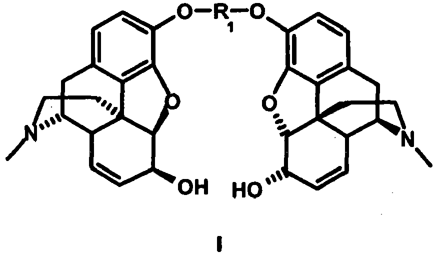

dans laquelle :
R₁ représente un groupement carbonyle, COR₅CO où R₅ représente un groupe (C₁-C₄)alcane-diyle, (C₂-C₄)alcène-diyle, (C₂-C₄)alcyne-diyle, hétéro(Cₓ₁-C_{y1})alcane-diyle, hétérocyclo(C₃-C₆)alcane-diyle, (C₅-C₁₄)aréne-diyle, hétéro(C₄-C₁₀)arène-diyle bi(C₁₀-C₁₆)arène-oxyde-diyle ou bi(C₁₀-C₁₆)arène-diyle, SO₂R₆SO₂ où R₆ représente un groupe (C₁-C₄)alcane-diyle, (C₂-C₄)alcène-diyle, (C₂-C₄)alcyne-diyle, hétéro(C₁-C₄)alcane-diyle, hétérocyclo(C₃-C₆)alcane-diyle, (C₅-C₁₄)arène-diyle, hétéro(C₄-C₁₀)arène-diyle, bi(C₁₀-C₁₆)arène-oxyde-diyle ou bi(C₁₀-C₁₆)arène-diyle.

Parmi les composés de formule (I), on peut citer en particulier :
- le téréphtalate de dimorphin-3-yle,
- l'isophtalate de dimorphin-3-yle,
- le phtalate de dimorphin-3-yle,
- le fumarate de dimorphin-3-yle,
- le benzène-1,2-disulfonate de dimorphin-3-yle,
- le benzène-1,3-disulfonate de dimorphin-3-yle,
- le thiophène-2,5-dicarboxylate de dimorphin-3-yle,
- le naphtalène-2,7-dicarboxylate de dimorphin-3-yle,
- le 4,4'-oxybenzoate de dimorphin-3-yle,
- le biphényl-4,4'-dicarboxylate de dimorphin-3-yle, et
- le carbonate de dimorphin-3-yle.

Le composé de formule (I) tel que défini précédemment peut être préparé selon différents procédés notamment par estérification du groupe phénol de la morphine par un acide dicarboxylique et élimination de l'eau par distillation azéotropique.

Il peut être aussi préparé selon le procédé décrit ci-dessous.

Selon un mode de réalisation particulier du procédé de la présente invention, celui-ci comprend préalablement à l'étape (i), les étapes consistant à faire réagir un composé de formule R₁Cl₂ dans laquelle R₁ est tel que défini précédemment, avec de la morphine en milieu diphasique comprenant au moins de l'eau, un agent basique fort et un solvant aromatique non substitué ou substitué par un ou plusieurs substituants choisis dans le groupe constitué par un atome d'halogène, un groupe (C₁-C₄)alkyle et un groupe (C₁-C₄)alkyloxy, ledit solvant présentant un point de fusion inférieur ou égal à -20°C.

Dans ce mode de réalisation de préparation de composé de formule (I), la morphine est introduite de préférence en excès par rapport au composé de formule R₁Cl₂, par exemple en un rapport molaire de 2,2 moles de morphine pour 1 mole de composé R₁Cl₂.

L'agent basique fort utilisé peut être de la soude.

Parmi les solvants pouvant être utilisés dans le procédé tels que définis ci-dessus, on peut citer en particulier le chlorobenzène, le toluène, le 1,2-dichlorobenzène, le 1,3,5-trifluorobenzène et le mésitylène. Avantageusement, on utilise le chlorobenzène qui assure la meilleure solubilisation des réactifs.

Avantageusement, on prépare d'abord le mélange de morphine et d'eau auquel on ajoute l'agent basique fort en quantité permettant d'obtenir un pH supérieur ou égal à 10. Le mélange est agité jusqu'à obtention d'une solution homogène. A cette solution homogène sont ajoutés le solvant et le composé de formule R₁Cl₂, de préférence lentement et sous forte agitation de façon à permettre le transfert de phase.

La présente invention a également pour objet les composés répondant à la formule (I).

Ces composés sont utiles comme intermédiaires de synthèse du M6G ou de ses dérivés.

### Composé de formule (II)

Dérivé d'acide glucuronique répondant à la formule (II) suivante : 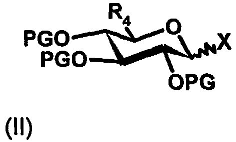 dans laquelle :
PG représente un groupe acétyle, isobutyryle, benzoyle ou pivaloyle,
X représente un groupe trihalogénoacétamidate, et
R₄ représente un groupe (Cₓ₁-C_{y1})alkylcarboxylate.

Parmi les dérivés d'acide glucuronique de formule (II), on peut citer en particulier ceux présentant une ou plusieurs des caractéristiques suivantes :
- PG représente un groupe acétyle,
- X représente un groupe -OCNHCl₃ ou un groupe -OCNPhCF₃, et
- R₄ représente un groupe méthylcarboxylate.

Selon un mode de réalisation particulier du procédé selon l'invention, le dérivé d'acide glucuronique de formule (II) est le trichloroacétimidate 2,3,4-tri-*O*-acétyl-α-D-glucopyranosyluronate de méthyle.
Le composé de formule (II) peut être préparé selon différents procédés bien connus de l'homme du métier.
Par exemple le trichloroacétimidate 2,3,4-tri-*O*-acétyl-α-D-glucopyranosyluronate de méthyle peut être synthétisé selon le procédé décrit dans le schéma 1 suivant :

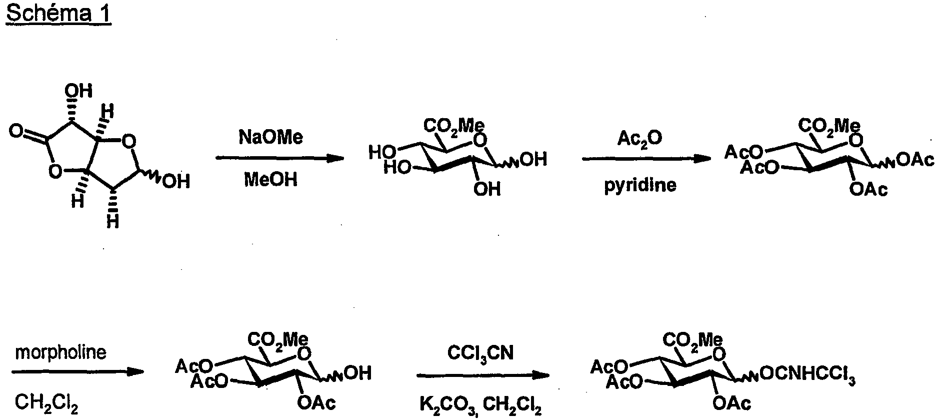

De tels procédés de synthèse sont notamment décrits dans Chem.Pharm. Bull. 53 (6) 684-687 (2005) pour le trichloroacétimidate 2,3,4-tri-*O*-acétyl-α-D-glucopyranosyluronate dans J.Chem. Soc. Perkin trans. 1 1995, pour le dérivé tri-O pivaloyl et dans Liebigs Ann. Chem. 1983, 570-574 pour le dérivé tri-O-benzoate.

### Paramètres de l'étape (i)

Comme indiqué précédemment, dans le procédé de l'invention, à l'étape (i) on fait réagir un composé de formule (I) avec un dérivé d'acide glucuronique de formule (II) en présence d'un solvant aromatique et de trifluorométhanesulfonyle de triméthylsilane.

Parmi les solvants aromatiques tels que définis précédemment pouvant être utilisés, on peut citer en particulier le chlorobenzène, le toluène, le 1,2-dichlorobenzène, le 1,3,5-trifluorobenzène et le mésitylène. Le chlorobenzène est en particulier avantageusement utilisé à la fois à l'étape (i) et lors de la préparation du composé de formule (I) comme indiqué précédemment.

Selon un mode de réalisation particulier, le rapport molaire dudit dérivé de formule (II) audit composé de formule (I) est compris entre 2 et 5 et est en particulier de 4.

La réaction est effectuée en présence d'un acide faible de Lewis, le trifluorométhanesulfonyle de triméthylsilane (TMSOTf).

Le rapport molaire du TMSOTf au composé de formule (I) est compris entre 2,2 et 20 et est en particulier de 3,1.

Selon un mode de réalisation particulier, le TMSOTf est introduit en deux temps : une première partie est introduite dans la solution du produit de formule (I) dans le solvant aromatique préalablement à l'addition du dérivé d'acide glucuronique, afin de salifier les deux azotes du composé de formule (I), puis la partie restante est introduite après l'addition du dérivé d'acide glucuronique afin d'assurer la O-glycosylation.

La mise en oeuvre de l'étape (i) conduit à la formation de composé répondant à la formule (III) suivante : 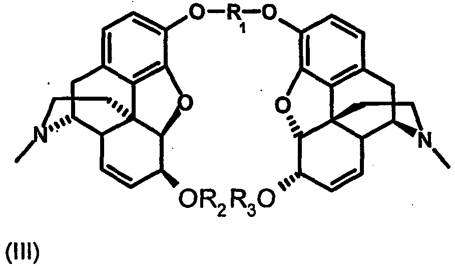 dans laquelle :
R₁ est tel que défini précédemment,
R₂ et R₃ représentent indépendamment un groupe PG tel que défini précédemment ou un groupe répondant à la formule (IV) suivante : 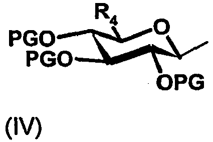 dans laquelle :
R₄ et PG sont tels que définis précédemment,
sous réserve que l'un au moins de R₂ et R₃ représente un groupe de formule (IV).

La présente invention a également pour objet les composés répondant à la formule (III). Ces composés sont utiles comme intermédiaires de synthèse du M6G ou de ses dérivés.

De préférence, R₂ et R₃ représentent tous deux un groupe de formule (IV).

Parmi les composés de formule (III) objets de l'invention, un premier groupe de composés présentent une ou plusieurs des caractéristiques suivantes :
- R₁ représente un groupement téréphtaloyle,
- au moins l'un de R₂ et R₃ représente un groupe de formule (IV) dans lequel R₄ est un groupe 2,3,4-tri-O-acétyl-β-D-glucuropryranosyluronate de méthyle et PG est un groupe acétyle.

Parmi ces composés, on peut citer en particulier :
- le téréphtalate de 6-*O*-acétylmorphin-3-yle et de 6-*O*-(2,3,4-tri-*O*-acétyl-β-D-glucopyranosyluronate de méthyle)morphin-3-yle, et
- le téréphtalate de di 6-*O*-(2,3,4-tri-*O*-acétyl-β-D-glucopyranosyluronate de méthyle)morphin-3-yle.

### Paramètres de l'étape (ii)

Comme indiqué précédemment, on fait réagir le produit obtenu à l'étape (i), à savoir le composé de formule (III), avec un agent basique fort.

Selon un mode de réalisation particulier, préalablement à l'addition de l'agent basique fort, le solvant aromatique est éliminé, selon des méthodes connues de l'homme du métier par exemple par extraction de la phase organique éventuellement complétée par évaporation sous pression réduite.

Généralement le composé de formule (III) est ensuite dissout dans un mélange hydro-alcoolique par exemple dans un mélange méthanol/eau, dans un rapport allant de 20/80 à 80/20, sous agitation, jusqu'à obtention d'un mélange homogène.

Ce mélange est généralement refroidi à une température inférieure ou égale à 5°C.

L'agent basique fort est ensuite introduit dans le mélange généralement en quantité permettant d'obtenir un pH supérieur ou égal à 10 et en particulier supérieur ou égal à 12,5, de préférence en maintenant la température à 5°C au plus.

Selon un mode de réalisation particulier, l'agent basique fort est de la soude.

Le mélange obtenu peut être ensuite chauffé par exemple à 20°C pendant un temps suffisant pour achever la réaction par exemple pendant une heure.

Lorsque l'on souhaite disposer du M6G ou de l'un de ses dérivés sous forme de base, le mélange, préalablement refroidi par exemple à une température inférieure ou égale à 5°C, est acidifié de sorte à ce qu'il présente un pH inférieur au pKₐ du produit à synthétiser, par exemple à un pH de 5,6. Cette acidification peut être en particulier effectuée par addition d'acide chlorhydrique.

Le mélange obtenu peut être ensuite chauffé par exemple à 20°C pendant un temps suffisant pour achever la réaction par exemple pendant 30 minutes.

### Paramètres de l'étape (iii)

Le produit obtenu à l'issue de l'étape (ii) peut être récupéré tel quel, c'est-à-dire sous forme brute, par exemple par filtration puis concentration sous vide du filtrat.

Avantageusement, il peut être récupéré sous forme purifiée, ce qui peut être réalisé selon toute méthode de purification connue de l'homme du métier en particulier par dessalage, le cas échéant suivi par une ou plusieurs étapes d'adsorption et de désorption sur résines échangeuses d'ions puis éventuellement par un ou plusieurs cycles de dissolution/évaporation/cristallisation.

Afin de réduire la teneur en sel en vue notamment d'éliminer l'acétate de sodium et le téréphtalate de sodium résiduels, on peut par exemple remettre le filtrat en suspension dans de l'alcool en particulier dans du méthanol dans des conditions permettant la dissolution du M6G ou de ses dérivés par exemple à 50°C pendant 3 heures, puis filtrer le mélange obtenu pour éliminer les particules solides et sécher le résidu obtenu par exemple par évaporation sous pression réduite.

La purification peut être poursuivie à l'aide de résines échangeuses d'ions. Selon un mode de réalisation particulier, le résidu obtenu précédemment est remis en suspension dans de l'eau déminéralisée, puis la suspension est acidifiée à un pH compris entre 2 et 4 et en particulier supérieur ou égal à 3, par exemple par addition d'acide sulfurique, filtrée et le filtrat est mis en contact avec une résine cationique dans des conditions permettant l'adsorption du M6G ou de son dérivé par exemple sous agitation à 20°C pendant 30 minutes, puis est filtrée. On répète ces opérations jusqu'à ce que le filtrat soit épuisé en M6G ou en son dérivé. Les résines sont ensuite désorbées avec une solution basique par exemple à l'aide d'ammoniaque. La solution basique obtenue est acidifiée à un pH compris entre 5 et 7 et en particulier de 6 puis séchée par exemple par évaporation sous pression réduite.

Enfin, ce résidu est remis en suspension dans un mélange hydro-alcoolique par exemple dans un mélange méthanol/eau, dans un rapport allant de 20/80 à 80/20, chauffé dans des conditions permettant sa dissolution totale par exemple à reflux pendant 30 minutes puis le mélange homogène est refroidi lentement par exemple jusqu'à O°C en 2 heures, les premiers cristaux apparaissant à 35°C.

Les cristaux sont isolés par exemple sur verre fritté, lavés par exemple à l'aide de méthanol, et finalement séchés par exemple par chauffage et évaporation sous vide.

L'invention est illustrée de manière non limitative par les exemples ci-dessous.

### EXEMPLES

### Synthèse

Le schéma 2 décrit la synthèse des composés des composés intermédiaires de formule (I) et (III) ainsi que du M6G et de ses dérivés.

Dans le schéma 2, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'Homme du métier.

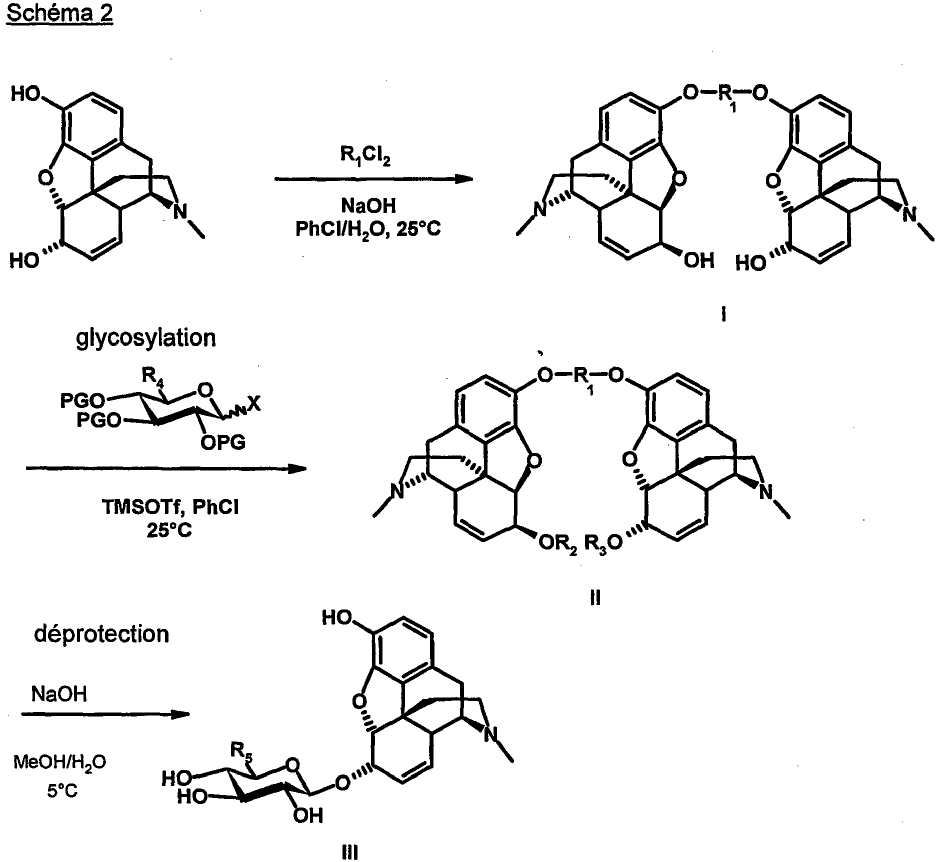

Les exemples suivants décrivent la préparation de certains composés conformes à l'invention. Ces exemples ne sont pas limitatifs et ne font qu'illustrer la présente invention.

### Préparation de composés de formule (I)

### Exemple 1- Téréphtalate de dimorphin-3-yle

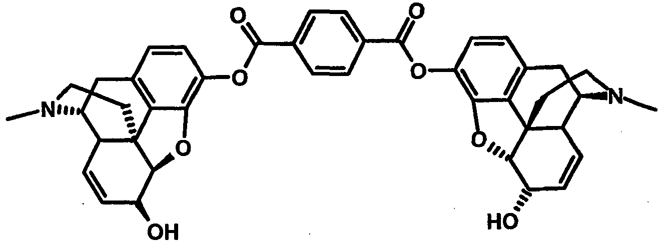

A température ambiante, à une solution de morphine monohydrate (40,0 g, 0,132 mol) dans de la soude 0,66N (300 mL, 0,198 mol) et du chlorobenzène (300 mL) est ajouté du chlorure de téréphtaloyle (12,0 g, 0,0594 mol) par petites portions en 2,5 h. Le milieu réactionnel est agité 15 min après la fin de l'ajout.

Le précipité formé est filtré et réempaté dans un mélange chlorobenzène/soude 0,66N (300 mL/300 mL) puis lavé avec de l'eau (3 x 250 mL) pour obtenir le téréphtalate de dimorphin-3-yle sous forme de cristaux blancs (38,2 g, 92%).

**RMN ¹H** (300 MHz, CDCl₃) δ 8,30 (s, 4H, *CH-téréphtalate*), 6,87 (d, 2H, *J* 8,0 Hz, H-1), 6,67 (d, 2H, *J* 8,0 Hz, H-2), 5,83 (m, 2H, H-8), 5,32 (m, 2H, H-7), 4,95 (d, 2H, *J* 6,0 Hz, H-5), 4,20 (m, 2H, H-6), 3,40 (m, 2H, H-9), 3,10 (m, 2H, H-10a), 2,74 (m, 2H, H-14), 2,67-2,61 (m, 2H, H-16a), 2,47 (s, 6H, NC*H*₃), 2,42-2,31 (m, 4H, H-10b, H-16b), 2,13-2,05 (m, 2H, H-15a), 1,96-1,92 (m, 2H, H-15b).

**RMN ¹³C** (75 MHz, CDCl₃) δ 163,3 (C=O), 148,8 (C-*ipso*), 134,3 (C-8), 130,5 (CH-*téréphtalate*), 129,7, 128,6 (C-*ipso*), 127,8 (C-7), 126,4 (C-*ipso*), 121,1 (C-1), 120,0 (C-2), 92,5 (C-5), 65,9 (C-6), 58,9 (C-9), 46,4 (C-16), 43,1 (NC*H*₃), 42,7 (C-13), 40,5 (C-14) 35,3 (C-15), 20,9 (C-10).

### Masse Haute Résolution (ES)

- Calculée pour C₄₂H₄₂N₂O₈ [M+H₂]²⁺ : m/z = 351,1471
- Trouvée : m/z = 351,1467

De la même façon, on a préparé les composés des exemples 2 à 11 suivants.

### Exemple 2- Isophtalate de dimorphin-3-yl

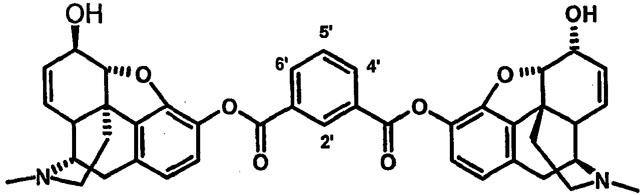

**RMN ¹H** (300 MHz, CDCl₃) δ 9,00 (t, 1H, *J* 1,5 Hz, H-2'), 8,44 (dd, *J* 1,5 Hz, *J* 8,0 Hz, H-4', H-6'), 7,66 (t, 1H, *J* 8,0 Hz, H-5'), 6,87 (d, 2H, *J* 8,0 Hz, H-1), 6,67 (d, 2H, *J* 8,0 Hz, H-2), 5,83 (m, 2H, H-8), 5,32 (m, 2H, H-7), 4,95 (d, 2H, *J* 6,0 Hz, H-5), 4,20 (m, 2H, H-6), 3,40 (m, 2H, H-9), 3,09 (m, 2H, H-10a), 2,72 (m, 2H, H-14), 2,66-2.60 (m, 2H, H-16a), 2,47 (s, 6H, NC*H*₃), 2,43-2,30 (m, 4H, H-10b, H-16b), 2,15-2,04 (m, 2H, H-15a), 1,95-1,91 (m, 2H, H-15b).
Masse (ionisation chimique) : [M+H]⁺=701,6

### Exemple 3- Phtalate de dimorphin-3-yle

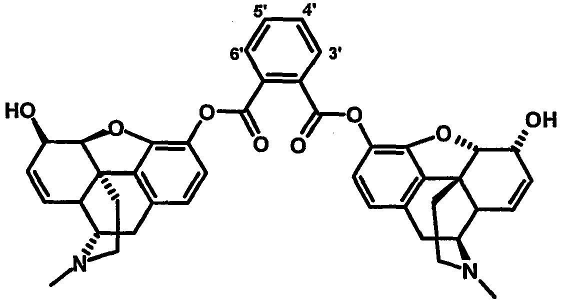

**RMN ¹H** (300 MHz, CDCl₃) δ 7,97 (dd, 2H, *J* 3,5Hz, *J* 6,0 Hz, H-3', H-6'), 7,67 (dd, *J* 3,5 Hz, *J* 6,0 Hz, H-4', H-5'), 6,87 (d, 2H, *J* 8,0 Hz, H-1), 6,59 (d, 2H, *J* 8,0 Hz, H-2), 5,74 (m, 2H, H-8), 5,29 (m, 2H, H-7), 4,81 (d, 2H, *J* 6,5 Hz, H-5), 4,15 (m, 2H, H-6), 3,37 (m, 2H, H-9), 3,06 (m, 2H, H-10a), 2,70 (m, 2H, H-14), 2,61-2,56 (m, 2H, H-16a), 2,44 (s, 6H, NC*H*₃), 2,42-2,27 (m, 4H, H-10b, H-16b), 2,08-2,05 (m, 2H, H-15a), 1,80 (m, 2H, H-15b).
Masse (ionisation chimique) : [M+H]⁺= 701,6

### Exemple 4- Fumarate de dimorphin-3-yle

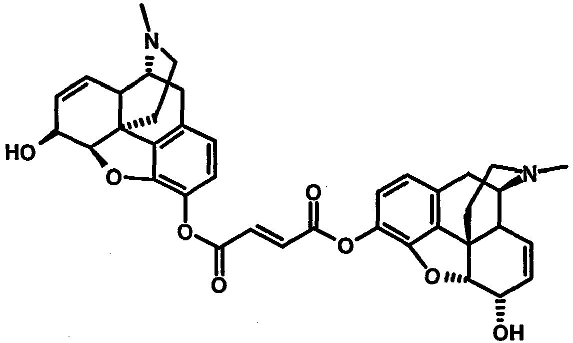

**RMN ¹H** (300 MHz, CDCl₃) 7,23 (s, 2H, C*H*COO), 6,81 (d, 2H, *J* 8,0 Hz, H-1), 6,64 (d, 2H, *J* 8,0 Hz, H-2), 5,78 (m, 2H, H-8), 5,30 (m, 2H, H-7), 4,95 (d, 2H, *J* 6,0 Hz, H-5), 4,19 (m, 2H, H-6), 3,42 (m, 2H, H-9), 3,08 (m, 2H, H-10a), 2,74 (m, 2H, H-14), 2,69-2,63 (m, 2H, H-16a), 2,47 (s, 6H, NC*H*₃), 2,42-2,30 (m, 4H, H-10b, H-16b), 2,16-2,06 (m, 2H, H-15a), 1,92 (m, 2H, H-15b).
Masse (ionisation chimique) : [M+H]⁺= 651,6

### Exemple 5- Benzène-1,2-disulfonate de dimorphin-3-yle

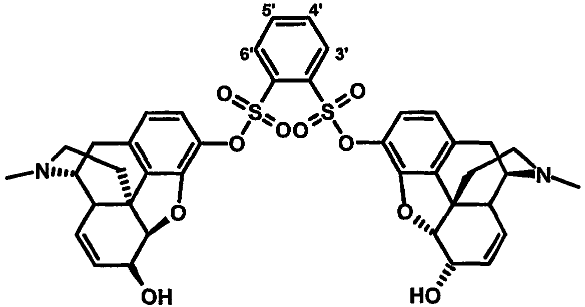

**RMN ¹H** (300 MHz, CDCl₃) δ 8,24 (dd, 2H, *J* 3,5 Hz, *J* 6,0 Hz, H-3', H-6'), 7,77 (dd, *J* 3,5 Hz, *J* 6,0 Hz, H-4', H-5'), 6,79 (d, 2H, *J* 8,5 Hz, H-1), 6,51 (d, 2H, *J* 8,5 Hz, H-2), 5,69 (m, 2H, H-8), 5,22 (m, 2H, H-7), 4,81 (d, 2H, *J* 6,5 Hz, H-5), 4,15 (m, 2H, H-6), 3,35 (m, 2H, H-9), 3,02 (m, 2H, H-10a), 2,65 (m, 2H, H-14), 2,60-2,54 (m, 2H, H-16a), 2,41 (s, 6H, NC*H*₃), 2,40-2,22 (m, 4H, H-10b, H-16b), 2,05-2,00 (m, 2H, H-15a), 1,77-1,72 (m, 2H, H-15b).
Masse (ionisation chimique) : [M+H]⁺= 773,6

### Exemple 6- Benzène-1,3-disulfonate de dimorphin-3-yle

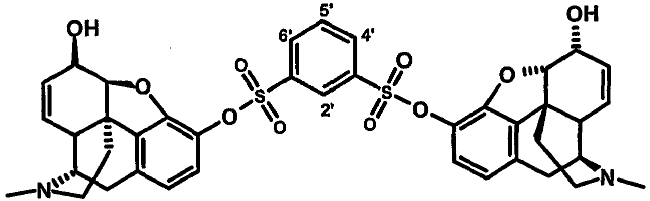

**RMN ¹H** (300 MHz, CDCl₃) δ 8,24 (m, 3H, H-2', H-4', H-6'), 7,78 (t, 1H, *J* 8,5 Hz, H-5'), 6,51 (m, 4H, H-1, H-2), 5,66 (m, 2H, H-8), 5,27 (m, 2H, H-7), 4,85 (d, 2H, *J 6,5* Hz, H-5), 4,15 (m, 2H, H-6), 3,34 (m, 2H, H-9), 3,03 (m, 2H, H-10a), 2,65 (m, 2H, H-14), 2,61-2,55 (m, 2H, H-16a), 2,42 (s, 6H, NC*H*₃), 2,35-2,24 (m, 4H, H-10b, H-16b), 2,09-1,99 (m, 2H, H-15a), 1,79-1,75 (m, 2H, H-15b).
Masse (ionisation chimique) : [M+H]⁺=773,6

### Exemple 7- Thiophène-2,5-dicarboxylate de dimorphin-3-yle

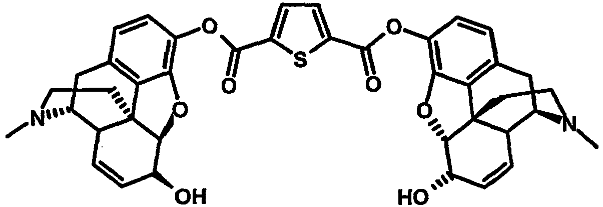

**RMN ¹H** (300 MHz, CDCl₃) δ 7,95 (s, 2H, *CH-thiophène*)*,* 6,86 (d, 1 H, *J* 8,0 Hz, H-1), 6,66 (d, 2H, *J* 8,0 Hz, H-2), 5,79 (m, 2H, H-8), 5,30 (m, 2H, H-7), 4,95 (d, 2H, *J* 6,5 Hz, H-5), 4,18 (m, 2H, H-6), 3,40 (m, 2H, H-9), 3,09 (m, 2H, H-10a), 2,75 (m, 2H, H-14), 2,68-2,62 (m, 2H, H-16a), 2,41 (s, 6H, NC*H*₃), 2,40-2,30 (m, 4H, H-10b, H-16b), 2,14-2,05 (m, 2H, H-15a), 1,95-1,90 (m, 2H, H-15b).
Masse (ionisation chimique) : [M+H]⁺= 708,6

### Exemple 8- Naphtalène-2,7-dicarboxylate de dimorphin-3-yle

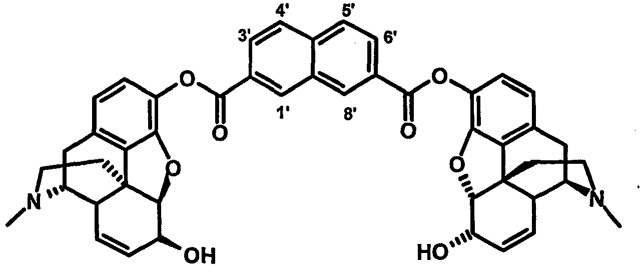

**RMN ¹H** (300 MHz, CDCl₃) δ 8,83 (s, 2H, H-1', H-8'), 8,27 (dd, *J* 1,5 Hz, *J* 8,5 Hz, 2H, H-3', H-6'), 8,08 (d, 2H, *J* 8,5 Hz, H-4', H-5'), 6,92 (d, 1 H, *J* 8,0 Hz, H-1), 6,69 (d, 2H, *J* 8,0 Hz, H-2), 5,84 (m, 2H, H-8), 5,34 (m, 2H, H-7), 4,95 (d, 2H, *J* 6,5 Hz, H-5), 4,20 (m, 2H, H-6), 3,40 (m, 2H, H-9), 3,11 (m, 2H, H-10a), 2,74 (m, 2H, H-14), 2,68-2,61 (m, 2H, H-16a), 2,47 (s, 6H, NC*H*₃), 2,44-2,32 (m, 4H, H-10b, H-16b), 2,14-2,05 (m, 2H, H-15a), 1,96-1,90 (m, 2H, H-15b).
Masse (ionisation chimique) : [M+H]⁺=751,6

### Exemple 9 - 4,4'-oxybenzoate de dimorphin-3-yle

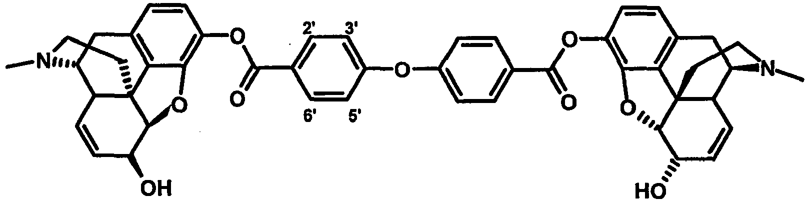

**RMN ¹H** (300 MHz, CDCl₃) δ 8,16 (d, 4H, *J* 8,5 Hz, H-2', H-6'), 7,14 (d, 4H, *J* 8,5 Hz, H-3', H-5'), 6,87 (d, 2H, *J* 8,5 Hz, H-1), 6,67 (d, 2H, *J* 8,5 Hz, H-2), 5,82 (m, 2H, H-8), 5,31 (m, 2H, H-7), 4,95 (d, 2H, *J* 6,5 Hz, H-5), 4,20 (m, 2H, H-6), 3,40 (m, 2H, H-9), 3,10 (m, 2H, H-10a), 2,73 (m, 2H, H-14), 2,66-2,61 (m, 2H, H-16a), 2,42 (s, 6H, NC*H*₃), 2,40-2,30 (m, 4H, H-10b, H-16b), 2,18-2,10 (m, 2H, H-15a), 1,95-1,90 (m, 2H, H-15b).
Masse (ionisation chimique) : [M+H]⁺= 793,5

### Exemple 10- Biphényl-4,4'-dicarboxylate de dimorphin-3-yle

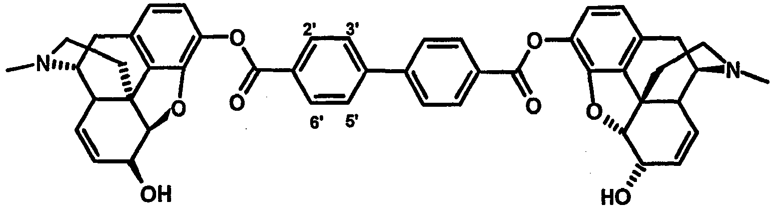

**RMN ¹H** (300 MHz, CDCl₃) δ 8,25 (d, 4H, *J* 8,0 Hz, H-2', H-6'), 7,79 (d, 4H, *J* 8,0 Hz, H-3', H-5'), 6,90 (d, 2H, *J* 8,0 Hz, H-1), 6,68 (d, 2H, *J* 8,0 Hz, H-2), 5,84 (m, 2H, H-8), 5,32 (m, 2H, H-7), 4,95 (d, 2H, *J* 6,5 Hz, H-5), 4,22 (m, 2H, H-6), 3,41 (m, 2H, H-9), 3,11 (m, 2H, H-10a), 2,74 (m, 2H, H-14), 2,68-2,63 (m, 2H, H-16a), 2,43 (s, 6H, NC*H*₃), 2,42-2,32 (m, 4H, H-10b, H-16b), 2,17-2,11 (m, 2H, H-15a), 1,95-1,90 (m, 2H, H-15b).
Masse (ionisation chimique) : [M+H]⁺= 777,5

### Exemple 11- Carbonate de dimorphin-3-yle

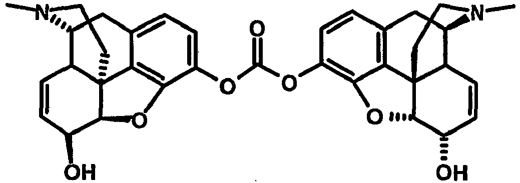

**RMN ¹H** (300 MHz, CDCl₃) δ 6,88 (d, 2H, J 8,0 Hz, H-1), 6,59 (d, 2H, J 8,0 Hz, H-2), 5,70 (m, 2H,H-8), 5,23 (m, 2H, H-7), 4,97 (d, 2H, J 6,5 Hz, H-5), 4,15 (m, 2H, H-6), 3,36 (m, 2H, H-9), 3,11 (m, 2H, H-10a), 2,69 (m, 2H, H-14), 2,68-2,60 (m, 2H, H-16a), 2,43 (s, 6H, NCH₃), 2,40-2,27 (m, 4H, H-10b, H-16b), 2,10-2,04 (m, 2H, H-15a), 1,94-1,90 (m, 2H, H-15b).
Masse (ionisation chimique) : [M+H]⁺= 597,5

### Glycosylation

A température ambiante, à une solution de téréphtalate de dimorphin-3-yle (50 mg, 0,071 mmol) dans du chlorobenzène (4 mL) est ajouté du TMSOTf (27 µL, 0,15 mmol). Le mélange réactionnel est agité pendant 3 min puis sont ajoutés du trichloroacétimidate de 2,3,4-tri-*O*-acétyl-α-D-glucopyranosyluronate de méthyle (171 mg, 0,36 mmol) puis du TMSOTf (13 µL, 0,071 mmol).

Le milieu réactionnel est agité 0,5 h à température ambiante.

Du NaHCO₃ (100 mg) est ajouté puis du CH₂Cl₂ (5 mL) et de l'eau (5 mL). La phase organique est séparée et séchée sur Na₂SO₄.

Le solvant est éliminé sous pression réduite. Un mélange de trois produits : téréphtalate de di 6-*O*-acétylmorphin-3-yle, téréphtalate de 6-*O*-acétylmorphin-3-yle et de 6-*O*-(2,3,4-tri-*O*-acétyl-β-D-glucopyranosyluronate de méthyle)morphin-3-yle et téréphtalate de di 6-*O*-(2,3,4-tri-*O*-acétyl-β-D-glucopyranosyluronate de méthyle)morphin-3-yle en proportions 7:30:63 est obtenu.

Une purification par chromatographie préparative en phase inverse (gradient (H₂O+0,1%TFA)-CH₃CN de 95:5 à 20:80) permet d'isoler les trois espèces.

### Exemple 12- Téréphtalate de di 6-O-acétylmorphin-3-yle

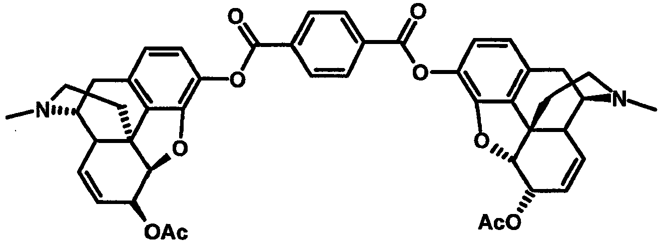

**RMN ¹H** (300 MHz, CDCl₃) δ 8,30 (s, 4H, *CH-téréphtalate*), 6,91 (d, 2H, *J* 8,0 Hz, H-1), 6,66 (d, 2H, *J* 8,0 Hz, H-2), 5,67 (m, 2H, H-8), 5,46 (m, 2H, H-7), 5,15 (m, 4H, H-5, H-6), 3,43 (m, 2H, H-9), 3,10 (m, 2H, H-10a), 2,81 (m, 2H, H-14), 2,67 (m, 2H, H-16a), 2,47 (s, 6H, NC*H*₃), 2,43-2,34 (m, 4H, H-10b, H-16b), 2,14-1,91 (m, 10H, C*H*₃CO, H-15).

**RMN ¹³C** (75 MHz, CDCl₃) δ 170,4, 163,2 (C=O), 149,5, 133,6, 132,5, 131,8, 131,6 (*C-ipso*), 130,3 (*CH-téréphtalate*), 129,2 (C-7), 128,7 (C-8), 121,9 (C-1), 119,5 (C-2), 88,7 (C-5), 68,0 (C-6), 58,9 (C-9), 46,5 (C-16), 42,8 (NC*H*₃), 42,7 (C-13), 40,3 (C-14) 35,0 (C-15), 20,8 (C-10), 20,6 (C*H*₃CO).

### Masse Haute Résolution (ES)

- Calculée pour C₄₆H₄₆N₂O₁₀ [M+H₂]²⁺ : m/z = 393,1576
- Trouvée : m/z = 393,1560

### Exemple 13- Téréphtalate de 6-O-acétylmorphin-3-yle et de 6-O-(2,3,4-tri-O-acétyl-β-D-glucopyranosyluronate de méthyle)morphin-3-yle

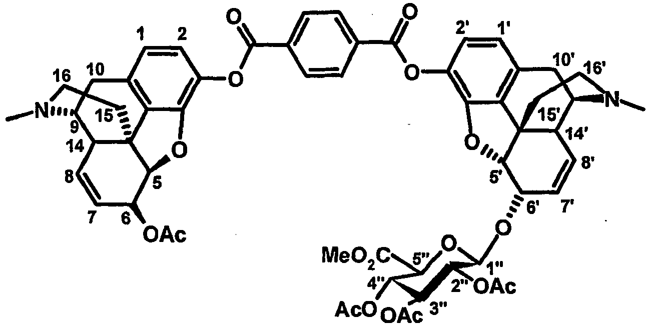

**RMN ¹H** (300 MHz, CDCl₃) δ 8,31 (m, 4H, *CH-téréphtalate),* 6,89 (m, 2H, H-1, H-1'), 6,65 (m, 2H, H-2, H-2'), 5,77 (m, 1 H, H-8'), 5,68 (m, 1 H, H-8), 5,46 (m, 1 H, H-7), 5,33 (m, 1 H, H-7'), 5,19 (m, 2H, H-3", H-4"), 5,15 (m, 1 H, H-6), 4,96 (m, 3H, H-2", H-5, H-5'), 4,86 (d, 1H, *J* 7.5 Hz, H-1"), 4,31 (m, 1 H, H-6'), 4,05 (m, 1 H, H-5"), 3,72 (s, 3H, *OCH₃),* 3,48 (m, 2H, H-9, H-9'), 3,11 (m, 2H, H-10a, H-10a'), 2,85-2,65 (m, 4H, H-14, H-14', H-16a, H-16a'), 2,52-2,37 (m, 10H, NC*H*₃, H-10b, H-10b', H-16b, H-16b'), 2,15-1,85 (m, 13H, H-15, H-15', C*H*₃CO), 1,73 (s, 3H, C*H*₃CO).

**RMN ¹³C** (75 MHz, CDCl₃) δ 170,4, 170,1, 169,4, 169,0, 167,3, 163,6, 163,3 (C=O), 150,4, 149,5 133,7, 133,6 (C-*ipso*), 132,0, 131,6, 130,8, 130,6, 130,3 129,0, 128,9 (*CH-téréphtalate,* C-8, C-8', C-7, C-7', C-*ipso*), 122,2, 122,0 (C-1, C-1'), 119,6, 119,3 (C-2, C-2'), 99,3 (C-1"), 89,8, 88,7 (C-5, C-5'), 73,7 (C-6'), 72,7 (C-5"), 71,8 (C-3" ou C-4"), 71,0 (C-2"), 69,4 (C-3" ou C-4"), 67,9 (C-6), 59,0, 58,8 (C-9, C-9'), 46,6, 46,3 (C-16, C-16'), 42,8 *(NCH₃),* 40,6, 40,2 (C-14, C-14') 35,2, 34,9 (C-15, C-15'), 21,2, 21,0, 20,7, 20,6, 20,5, 20,4 (C-10, C-10' C*H*₃CO).

### Masse Haute Résolution (ES)

- Calculée pour C₅₇H₆₀N₂O₁₈ [M+H₂]²⁺ : m/z = 530,1921
- Trouvée : m/z = 530,1918

### Exemple 14- Téréphtalate de di 6-O-(2,3,4-tri-O-acétyl-β-D-glucopyranosyluronate de méthyle)morphin-3-yle

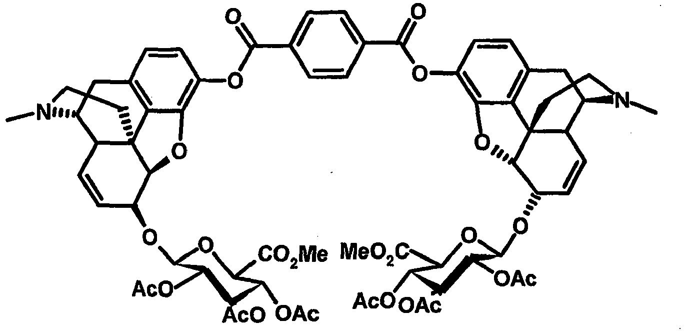

**RMN ¹H** (300 MHz, CDCl₃) δ 8,34 (s, 4H, *CH-téréphtatate*), 6,90 (d, 2H, *J* 8,0 Hz, H-1), 6,63 (d, 2H, *J* 8,0 Hz, H-2), 5,77 (m, 2H, H-8), 5,34 (m, 2H, H-7), 5,23 (m, 4H, H-3', H-4'), 5,02-4,94 (m, 4H, H-2', H-5), 4,86 (d, 2H, *J* 7,5 Hz, H-1'), 4,32 (m, 2H, H-6), 4,06 (d, 2H, *J* 9,5 Hz, H-5'), 3,72 (s, 6H, OC*H*₃), 3,48 (m, 2H, H-9), 3,11 (m, 2H, H-10a), 2,75-2.60 (m, 4H, H-14, H-16a), 2,51-2,25 (m, 10H, NC*H*₃, H-10b, H-16b), 2,16-1,90 (m, 16H, H-15, C*H*₃CO), 1,75 (s, 6H, C*H*₃CO).

**RMN ¹³C** (75 MHz, CDCl₃) δ 170,0, 169,4, 169,3, 167,4, 163,9 (C=O), 150,4, 133,7, 132,5, 131,7, 131,4 (C-*ipso*), 130,6 (*CH-téréphtatate,* C-8), 128,9 (C-7), 122,0 (C-1), 119,3 (C-2), 99,2 (C-1'), 88,7 (C-5), 73,6 (C-6), 72,9 (C-5'), 71,7 (C-3' ou C-4'), 70,9 (C-2'), 69,4 (C-3' ou C-4'), 58,8 (C-9), 52,9 (OC*H*₃), 46,2 (C-16), 43,1 (NC*H*₃), 41,1 (C-14), 35,7 (C-1 5), 21,0 (C-10), 20,6, 20,5, 20,4 (C*H*₃CO).

### Masse Haute Résolution (ES)

- Calculée pour C₆₈H₇₄N₂O₂₆ [M+H₂]²⁺ : m/z = 667,2265
- Trouvée : m/z = 667,2253

### Saponification des intermédiaires issus du couplage O-glycosidigue

Après traitement et extraction de la phase organique (chlorobenzène) issue du couplage, le chlorobenzène est évaporé sous pression réduite (15 mbar) pour obtenir une huile brunâtre (m= 47, 4 g). A cette huile est ajoutée un mélange de méthanol (140 ml) et d'eau déminéralisée (35ml) à 30°C, sous agitation jusqu'à obtention d'un mélange homogène. Le mélange est ensuite refroidi à -5°C.

A ce mélange, 62,5 ml d'une solution de soude concentrée (30% m/m) sont ajoutés en ne dépassant pas une température de 5°C dans le réacteur. Ce mélange (pH= 12,72) est ensuite chauffé à 20°C pendant 1 h sous azote, puis refroidi à -3°C.

Au mélange ainsi obtenu, 37 ml d'une solution d'acide chlorhydrique (HCl 37%) sont ajoutés (pH = 5,6). Le mélange est chauffé à 20°C et maintenu à cette température pendant 30 mn (pH stable à 5,6). Le mélange est ensuite filtré et le filtrat obtenu est concentré sous vide (15 mbar) pour obtenir un extrait sec de 67,0 g.

### Purification du résidu issu de la saponification et acidification

Ce résidu est mis en suspension dans 500 ml de méthanol à 50C° pendant 3 h (dessalage) pour obtenir après filtration et évaporation sous pression réduite (15 mbar) un résidu de 30,8g comprenant un mélange de morphine analysée en H.P.L.C. (de l'ordre de 30%) et de M6G (de l'ordre de 70%).

Le résidu obtenu précédemment est mis en suspension dans 100 ml d'eau déminéralisée et la suspension obtenue est acidifiée à pH égal à 3,58 avec H2SO4 98 % (2 ml) puis filtrée.

Au filtrat, est ajoutée 6g de la résine commercialisée sous la dénomination « IRP 69 » par la société Rohm et Hass (dans un rapport pondéral de 3 par rapport au poids de M6G contenu selon l'estimation obtenue par HPLC). Le mélange hétérogène ainsi obtenu est maintenu sous agitation à 20 °C pendant 30 mn puis filtré.

L'opération est réitérée jusqu'à épuisement du M6G contenu.

Les résines obtenues sont désorbées avec de l'ammoniaque dilué (3% NH4OH) et la solution basique obtenue (pH = 10,9) est neutralisée à un pH de l'ordre de 5 à 6 avec du HCl dilué.

La solution aqueuse acide obtenue est évaporée sous pression réduite (15mbar) pour obtenir un résidu sec de 5,6 g.

Il est identifié en H.P.L.C (teneur en M6G de l'ordre de 80%).

### Recristallisation du M6G

Le résidu sec précédent (5,55 g) est mis en suspension dans un mélange d'eau (166,5 ml) et de méthanol (277,5 ml). Le mélange est chauffé à reflux (90°C) pendant 30 mn puis refroidi jusqu'à 0°C en 2 hr. Les premiers cristaux se forment à 35°C.

Les cristaux sont isolés sur verre frité puis lavés avec 5 ml de méthanol. Après séchage à 80°C sous 15 mbar, 2,6 g de M6G pur (pureté organique >99%) sont isolés. 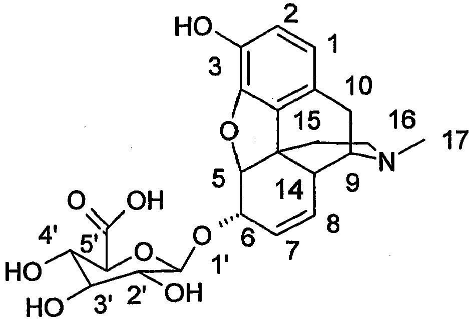 HPLC (Teneur en M6G) = 98,6%
Masse (ionisation chimique) = [M+ H] += 462,2
RMN 13C (75 MHz, CDCl3) (D2O/ Echange NaOD) δ(ppm +/- 0,01 ppm): 129,71, 127,42 (C1, C2) ; 155, 73 , 149,25 (C3, C4) ; 98,19 (C5) ; 111,12 (C1') ; 85,82 , 85,17, 82,94, 82,77, 81,40 (C6, C2', C3', C4', C5') ; 68,66 (C9) ; 55,76 (C16) ; 52,07 (C13) ; 50,79 (C17) ; 48,34 (C14); 42,80 (C15) ; 30,70 (C10) ; 185,30 (CO2H)

## Revendications

1. Procédé de préparation de morphine-6-glucuronide ou l'un de ses dérivés comprenant les étapes consistant :
(i) à faire réagir un composé répondant à la formule (I) suivante : 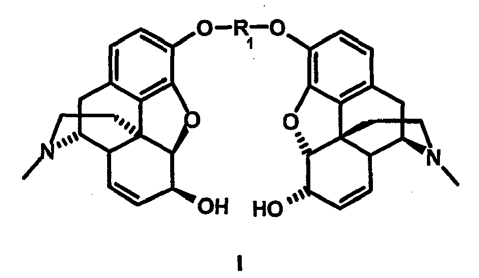 dans laquelle:
R₁ représente un groupement carbonyle, COR₅CO où R₅ représente un groupe (C₁-C₄)alcane-diyle, (C₂-C₄)alcène-diyle, (C₂-C₄)alcyne-diyle, hétéro(C₁-C₄)alcane-diyle, hétérocyclo(C₃-C₆)alcane-diyle, (C₅-C₁₄)arène-diyle, bi(C₁₀-C₁₆)arène-oxyde-diyle, bi(C₁₀-C₁₆)arène-diyle, ou hétéro(C₄-C₁₀)arène-diyle, SO₂R₆SO₂ où R₆ représente un groupe (C₁-C₄)alcane-diyle, (C₂-C₄)alcène-diyle, (C₂-C₄)alcyne-diyle, hétéro(C₁-C₄)alcane-diyle, hétérocyclo(C₃-C₆)alcane-diyle, (C₅-C₁₄)arène-diyle, hétéro(C₄-C₁₀)arène-diyle, bi(C₁₀-C₁₆)arène-oxyde-diyle ou bi(C₁₀-C₁₆)arène-diyle, avec un dérivé d'acide glucuronique répondant à la formule (II) suivante : 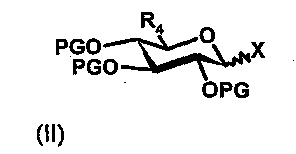
dans laquelle :
PG représente un groupe acétyle, isobutyryle, benzoyle ou pivaloyle,
X représente un groupe trihalogénoacétamidate, et
R₄ représente un groupe (C₁-C₄)alkylcarboxylate,
en présence :
- d'un solvant aromatique non substitué ou substitué par un ou plusieurs substituants choisis dans le groupe constitué par un atome d'halogène, un groupe (C₁-C₄)alkyle et un groupe (C₁-C₄)alkyloxy, ledit solvant présentant un point de fusion inférieur ou égal à - 20°C, et
- de trifluorométhanesulfonyle de triméthylsilane
(ii) à faire réagir le produit obtenu à l'étape (i) avec un agent basique fort, puis
(iii) à récupérer le produit obtenu à l'étape (ii).

2. Procédé selon la revendication 1, dans lequel préalablement à l'étape (i), on fait réagir un composé de formule R₁Cl₂ dans laquelle R₁ est tel que défini en revendication 1, avec de la morphine en milieu diphasique comprenant au moins de l'eau, un agent basique fort et un solvant aromatique non substitué ou substitué par un ou plusieurs substituants choisis dans le groupe constitué par un atome d'halogène, un groupe (C₁-C₄)alkyle et un groupe (C₁-C₄)alkyloxy, ledit solvant présentant un point de fusion inférieur ou égal à-20°C.

3. Procédé selon la revendication 1 ou 2, **caractérisé en ce que** ledit composé de formule (I) est choisi dans le groupe constitué par :
- le téréphtalate de dimorphin-3-yle,
- l'isophtalate de dimorphin-3-yle,
- le phtalate de dimorphin-3-yle,
- le fumarate de dimorphin-3-yle,
- le benzène-1,2-disulfonate de dimorphin-3-yle,
- le benzène-1,3-disulfonate de dimorphin-3-yle,
- le thiophène-2,5-dicarboxylate de dimorphin-3-yle,
- le naphtalène-2,7-dicarboxylate de dimorphin-3-yle,
- le 4,4'-oxybenzoate de dimorphin-3-yle,
- le biphényl-4,4'-dicarboxylate de dimorphin-3-yle, et
- le carbonate de dimorphin-3-yle.

4. Procédé selon l'une quelconque des revendications précédentes **caractérisé en ce que** ledit composé de formule (II) présente une ou plusieurs des caractéristiques suivantes:
- PG représente un groupe acétyle,
- X représente un groupe -OCNHCl₃ ou un groupe -OCNPhCF₃, et
- R₄ représente un groupe méthylcarboxylate.

5. Procédé selon l'une quelconque des revendications précédentes, **caractérisé en ce que** ledit composé de formule (II) est trichloroacétimidate 2,3,4-tri-*O*-acétyl-α-D-glucopyranosyluronate de méthyle.

6. Procédé selon l'une quelconque des revendications précédentes, **caractérisé en ce que** ledit solvant aromatique est choisi parmi le chlorobenzène, le toluène, le 1,2-dichlorobenzène, le 1,3,5-trifluorobenzène et le mésitylène.

7. Procédé selon l'une quelconque des revendications précédentes, **caractérisé en ce que** ledit agent basique fort est la soude.

8. Procédé selon l'une quelconque des revendications précédentes, **caractérisé en ce que** le rapport molaire dudit dérivé d'acide glucuronique de formule (II) audit composé de formule (I) est compris entre 2 et 5.

9. Procédé selon l'une quelconque des revendications précédentes, **caractérisé en ce que** le rapport molaire du trifluorométhanesulfonyle de triméthylsilane audit composé de formule (I) est compris entre 2,2 et 20.

10. Composé de formule 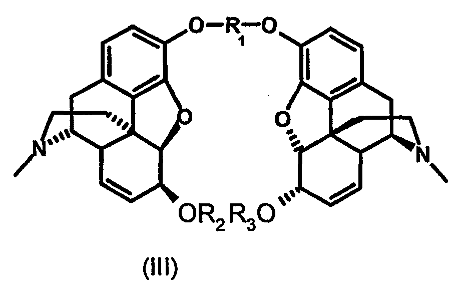 dans laquelle :
R₁ est tel que défini en revendication 1,
R₂ et R₃ représentent indépendamment un groupe PG tel que défini en revendication 1 ou un groupe de formule (IV) suivante : 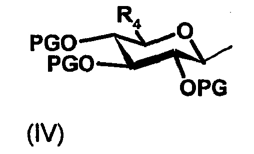
dans laquelle :
R₄ et PG sont tels que définis en revendication 1,
sous réserve que l'un au moins de R₂ et R₃ représente un groupe de formule (IV).

11. Composé selon la revendication 10, **caractérisé en ce qu'**il présente une ou plusieurs des caractéristiques suivantes :
- R₁ représente un groupement téréphtaloyle,
- au moins l'un de R₂ et R₃ représente un groupe de formule (IV) dans lequel R4 est un groupe 2,3,4-tri-O-acétyl-β-D-glucuropryranosyluronate de méthyle et PG est un groupe acétyle.

12. Composé selon la revendication 10 ou 11, **caractérisé en ce qu'**il est choisi dans le groupe constitué par :
- le téréphtalate de 6-*O*-acétylmorphin-3-yle et de 6-*O*-(2,3,4-tri-*O*-acétyl-β-D-glucopyranosyluronate de méthyle)morphin-3-yle, et
- le téréphtalate de di 6-*O*-(2,3,4-tri-*O*-acétyl-β-D-glucopyranosyluronate de méthyle)morphin-3-yle.

13. Composé de formule 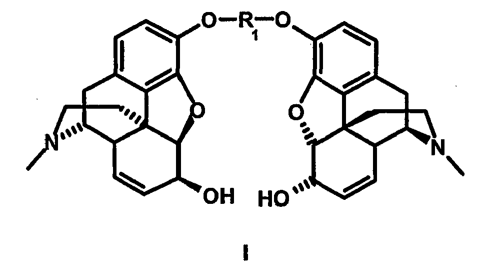 dans laquelle :
R₁ est tel que défini en revendication 1.

14. Composé selon la revendication 13, choisi dans le groupe constitué par:
- le téréphtalate de dimorphin-3-yle,
- l'isophtalate de dimorphin-3-yle,
- le phtalate de dimorphin-3-yle,
- le fumarate de dimorphin-3-yle,
- le benzène-1,2-disulfonate de dimorphin-3-yle,
- le benzène-1,3-disulfonate de dimorphin-3-yle,
- le thiophène-2,5-dicarboxylate de dimorphin-3-yle,
- le naphtalène-2,7-dicarboxylate de dimorphin-3-yle,
- le 4,4'-oxybenzoate de dimorphin-3-yle,
- le biphényl-4,4'-dicarboxylate de dimorphin-3-yle, et
- le carbonate de dimorphin-3-yle.

## Patentansprüche

1. Verfahren zur Herstellung von Morphin-6-glucuronid oder einem seiner Derivate, umfassend die folgenden Schritte:
(i) Umsetzen einer Verbindung der folgenden Formel (I) 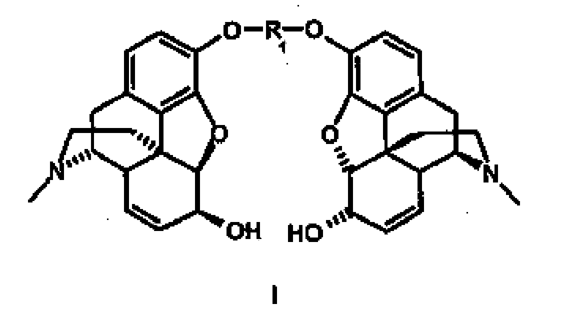 mit den folgenden Bedeutungen:
R₁ bedeutet eine Carbonylgruppe, COR₅CO, worin R₅ eine (C₁-C₄)-Alkandiyl-, (C₂-C₄)-Alkendiyl-, (C₂-C₄)-Alkindiyl-, Hetero-(C₁-C₄)-alkandiyl-, Heterocyclo-(C₃-C₆)-alkandiyl-, (C₅-C₁₄)-Arendiyl, Bi-(C₁₀-C₁₆)-arenoxiddiyl-, Bi-(C₁₀-C₁₆)-arendiyl- oder Hetero-(C₄-C₁₀)-Arendiylgruppe bedeutet, SO₂R₆SO₂, worin R₆ eine (C₁-C₄)-Alkandiyl-, (C₂-C₄)-Alkendiyl-, (C₂-C₄)-Alkindiyl-, Hetero-(C₁-C₄)-alkandiyl-, Heterocyclo-(C₃-C₆)-alkandiyl-, (C₅-C₁₄)-Arendiyl, Hetero-(C₄-C₁₀)-arendiyl-, Bi-(C₁₀-C₁₆)-arenoxiddiyl- oder Bi(C₁₀-C₁₆)-arendiylgruppe bedeutet, mit einem Glucuronsäurederivat der folgenden Formel (II): 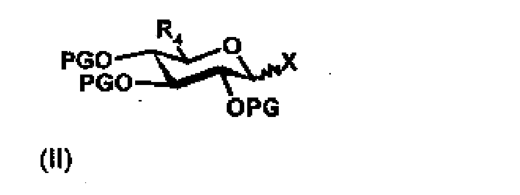 mit den folgenden Bedeutungen:
PG bedeutet eine Acetyl-, Isobutyryl-, Benzoyl- oder Pivaloylgruppe,
X bedeutet eine Trihalogenacetamidatgruppe und
R₄ bedeutet eine (C₁-C₄)-Alkylcarboxylatgruppe, in Gegenwart:
- eines aromatischen Lösungsmittels, das unsubstituiert oder durch einen oder mehrere Substituenten, ausgewählt aus der Gruppe bestehend aus einem Halogenatom, einer (C₁-C₄)-Alkylgruppe und einer (C₁-C₄)-Alkyloxygruppe substituiert ist, wobei das Lösungsmittel einen Schmelzpunkt von -20°C oder darunter aufweist, und
- von Trimethylsilyltrifluormethansulfonat,
(ii) Umsetzen des in Schritt (i) erhaltenen Produkts mit einer starken Base und dann
(iii) Gewinnen des in Schritt (ii) erhaltenen Produkts.

2. Verfahren nach Anspruch 1, in dem man vor Schritt (i) eine Verbindung der Formel R₁Cl2, in der R₁ wie in Anspruch 1 definiert ist, mit Morphin in einem diphasischen Medium umfassend zumindest Wasser, eine starke Base und ein aromatisches Lösungsmittel, das unsubstituiert oder durch einen oder mehrere Substituenten, ausgewählt aus der Gruppe bestehend aus einem Halogenatom, einer (C₁-C₄)-Alkylgruppe und einer (C₁-C₄)-Alkyloxygruppe substituiert ist, wobei das Lösungsmittel einen Schmelzpunkt von -20°C oder darunter aufweist, umsetzt.

3. Verfahren nach Anspruch 1 oder 2, **dadurch gekennzeichnet, dass** die Verbindung der Formel (I) aus der Gruppe bestehend aus den Folgenden ausgewählt ist:
- Dimorphin-3-ylterephthalat,
- Dimorphin-3-ylisophthalat,
- Dimorphin-3-ylphthalat,
- Dimorphin-3-ylfumarat,
- Dimorphin-3-ylbenzol-1,2-disulfonat,
- Dimorphin-3-ylbenzol-1,3-disulfonat,
- Dimorphin-3-ylthiophen-2,5-dicarboxylat,
- Dimorphin-3-yl-naphthalin-2,7-dicarboxylat,
- Dimorphin-3-yl-4,4'-oxybenzoat,
- Dimorphin-3-ylbiphenyl-4,4'-dicarboxylat und
- Dimorphin-3-ylcarbonat.

4. Verfahren nach einem der vorhergehenden Ansprüche, **dadurch gekennzeichnet, dass** die Verbindung der Formel (II) eines oder mehrere der folgenden Merkmale aufweist:
- PG bedeutet eine Acetylgruppe,
- X bedeutet eine -OCNHCl₃-Gruppe oder eine -OCNPhCF₃-Gruppe, und
- R₄ bedeutet eine Methylcarboxylatgruppe.

5. Verfahren nach einem der vorhergehenden Ansprüche, **dadurch gekennzeichnet, dass** es sich bei der Verbindung der Formel (II) um Methyl-2,3,4-tri-*O*-acetyl-α-D-glucopyranosyluronattrichloracetimidad handelt.

6. Verfahren nach einem der vorhergehenden Ansprüche, **dadurch gekennzeichnet, dass** das aromatische Lösungsmittel aus Chlorbenzol, Toluol, 1,2-Dichlorbenzol, 1,3,5-Trifluorbenzol und Mesitylen ausgewählt ist.

7. Verfahren nach einem der vorhergehenden Ansprüche, **dadurch gekennzeichnet, dass** es sich bei der starken Base um Natriumhydroxid handelt.

8. Verfahren nach einem der vorhergehenden Ansprüche, **dadurch gekennzeichnet, dass** das Molverhältnis des Glucuronsäurederivats der Formel (II) zu der Verbindung der Formel (I) zwischen 2 und 5 liegt.

9. Verfahren nach einem der vorhergehenden Ansprüche, **dadurch gekennzeichnet, dass** das Molverhältnis von Trimethylsilyltrifluormethansulfonat zu der Verbindung der Formel (I) zwischen 2,2 und 20 liegt.

10. Verbindung der Formel 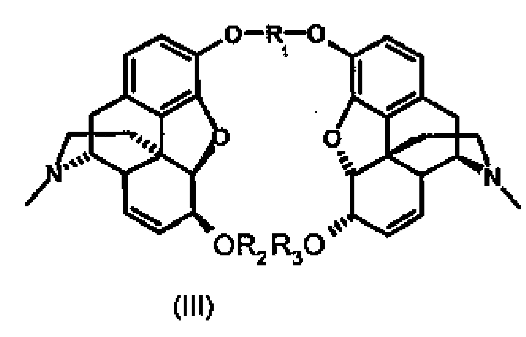 mit den folgenden Bedeutungen:
R₁ ist wie in Anspruch 1 definiert,
R₂ und R₃ bedeuten unabhängig eine PG-Gruppe wie in Anspruch 1 definiert oder eine Gruppe der folgenden Formel (IV): 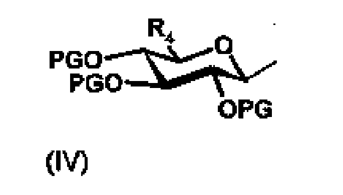
mit den folgenden Bedeutungen:
R₄ und PG sind wie in Anspruch 1 definiert,
mit der Maßgabe, dass mindestens eines von R₂ und R₃ eine Gruppe der Formel (IV) bedeutet.

11. Verbindung nach Anspruch 10, **dadurch gekennzeichnet, dass** sie eines oder mehrere der folgenden Merkmale aufweist:
- R₁ bedeutet eine Terephthaloylgruppe,
- mindestens eines von R₂ und R₃ bedeutet eine Gruppe der Formel (IV), worin R₄ eine Methyl-2,3,4-tri-O-acetyl-β-D-glucuropyranosyluronatgruppe bedeutet und PG eine Acetylgruppe bedeutet.

12. Verbindung nach Anspruch 10 oder 11, **dadurch gekennzeichnet, dass** sie aus der Gruppe bestehend aus den Folgenden ausgewählt ist:
- 6-*O*-Acetylmorphin-3-yl-6-*O*-(methyl-2,3,4-tri-*O*-acetyl-β-D-glucopyranosyluronat)morphin-3-ylterephthalat und
- Di[6-*O*-(methyl-2,3,4-tri-*O*-acetyl-β-D-glucopyranosyluronat)morphin-3-yl]terephthalat.

13. Verbindung der Formel 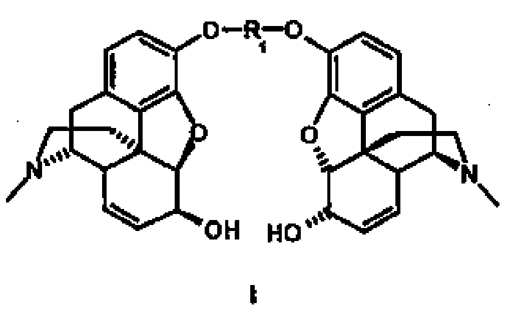 in der:
R₁ wie in Anspruch 1 definiert ist.

14. Verbindung nach Anspruch 13, ausgewählt aus der Gruppe bestehend aus:
- Dimorphin-3-ylterephthalat,
- Dimorphin-3-ylisophthalat,
- Dimorphin-3-ylphthalat,
- Dimorphin-3-ylfumarat,
- Dimorphin-3-ylbenzol-1,2-disulfonat,
- Dimorphin-3-ylbenzol-1,3-disulfonat,
- Dimorphin-3-ylthiophen-2,5-dicarboxylat,
- Dimorphin-3-yl-naphthalin-2,7-dicarboxylat,
- Dimorphin-3-yl-4,4'-oxybenzoat,
- Dimorphin-3-ylbiphenyl-4,4'-dicarboxylat und
- Dimorphin-3-ylcarbonat.

## Claims

1. Process for preparing morphine-6-glucuronide or a derivative thereof, comprising the steps consisting in:
(i) reacting a compound corresponding to formula (I) below: 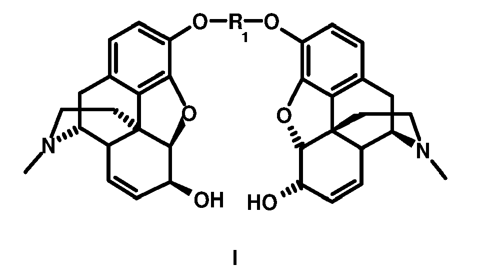 in which:
R₁ represents a carbonyl group, COR₅CO in which R₅ represents a group (C₁-C₄)alkane-diyl, (C₂-C₄)alkene-diyl, (C₂-C₄)alkyne-diyl, hetero(C₁-C₄)alkane-diyl, heterocyclo(C₃-C₆)alkane-diyl, (C₅-C₁₄)arene-diyl, bi(C₁₀-C₁₆)arene-oxide-diyl, bi(C₁₀-C₁₆)arene-diyl, or hetero(C₄-C₁₀)arene-diyl, SO₂R₆SO₂ in which R₆ represents a group (C₁-C₄)alkane-diyl, (C₂-C₄)alkene-diyl, (C₂-C₄)alkyne-diyl, hetero(C₁-C₄)alkane-diyl, heterocyclo(C₃-C₆)alkane-diyl, (C₅-C₁₄)arene-diyl, hetero(C₄-C₁₀)arene-diyl, bi(C₁₀-C₁₆)arene-oxide-diyl or bi(C₁₀-C₁₆)arene-diyl,
with a glucuronic acid derivative corresponding to formula (II) below: 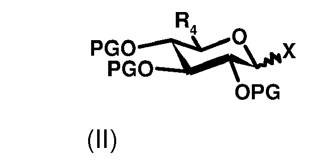
in which:
PG represents an acetyl, isobutyryl, benzoyl or pivaloyl group,
X represents a trihaloacetimidate group, and
R₄ represents a group (C₁-C₄)alkylcarboxylate,
in the presence:
- of an aromatic solvent that is unsubstituted or substituted with one or more substituents chosen from the group formed by a halogen atom, a group (C₁-C₄)alkyl and a group (C₁-C₄)alkyloxy, said solvent having a melting point of less than or equal to - 20°C, and
- of trimethylsilyl trifluoromethanesulfonate
(ii) in reacting the product obtained in step (i) with a strong basic agent, and then
(iii) in recovering the product obtained in step (ii).

2. Process according to Claim 1, in which, prior to step (i), a compound of formula R₁Cl₂ in which R₁ is as defined in Claim 1 is reacted with morphine in a two-phase medium comprising at least water, a strong basic agent and an aromatic solvent that is unsubstituted or substituted with one or more solvents chosen from the group formed by a halogen atom, a group (C₁-C₄)alkyl and a group (C₁-C₄)alkyloxy, said solvent having a melting point of less than or equal to -20°C.

3. Process according to Claim 1 or 2, **characterized in that** said compound of formula (I) is chosen from the group formed by:
- dimorphin-3-yl terephthalate,
- dimorphin-3-yl isophthalate,
- dimorphin-3-yl phthalate,
- dimorphin-3-yl fumarate,
- dimorphin-3-yl benzene-1,2-disulfonate,
- dimorphin-3-yl benzene-1,3-disulfonate,
- dimorphin-3-yl thiophene-2,5-dicarboxylate,
- dimorphin-3-yl naphthalene-2,7-dicarboxylate,
- dimorphin-3-yl 4,4'-oxybenzoate,
- dimorphin-3-yl biphenyl-4,4'-dicarboxylate, and
- dimorphin-3-yl carbonate.

4. Process according to any one of the preceding claims, **characterized in that** said compound of formula (II) has one or more of the following characteristics:
- PG represents an acetyl group,
- X represents a group -OCNHCl₃ or a group -OCNPhCF₃, and
- R₄ represents a methylcarboxylate group.

5. Process according to any one of the preceding claims, **characterized in that** said compound of formula (II) is methyl 2,3,4-tri-O-acetyl-α-D-glucopyranosyluronate trichloroacetimidate.

6. Process according to any one of the preceding claims, **characterized in that** said aromatic solvent is chosen from chlorobenzene, toluene, 1,2-dichlorobenzene, 1,3,5-trifluorobenzene and mesitylene.

7. Process according to any one of the preceding claims, **characterized in that** said strong basic agent is sodium hydroxide.

8. Process according to any one of the preceding claims, **characterized in that** the mole ratio of said glucuronic acid derivative of formula (II) to said compound of formula (I) is between 2 and 5.

9. Process according to any one of the preceding claims, **characterized in that** the mole ratio of the trimethylsilyl trifluoromethanesulfonate to said compound of formula (I) is between 2.2 and 20.

10. Compound of formula 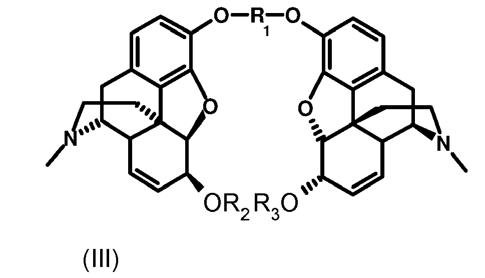 in which:
R₁ is as defined in Claim 1,
R₂ and R₃ independently represent a group PG as defined in Claim 1 or a group of formula (IV) below: 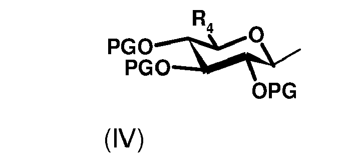
in which:
R₄ and PG are as defined in Claim 1,
with the proviso that at least one from among R₂ and R₃ represents a group of formula (IV).

11. Compound according to Claim 10, **characterized in that** it has one or more of the following characteristics:
- R₁ represents a terephthaloyl group,
- at least one from among R₂ and R₃ represents a group of formula (IV) in which R4 is a methyl 2,3,4-tri-O-acetyl-β-D-glucuropryranosyluronate group and PG is an acetyl group.

12. Compound according to Claim 10 or 11, **characterized in that** it is chosen from the group formed by:
- 6-*O*-acetylmorphin-3-yl 6-*O*-(methyl 2,3,4-tri-*O*-acetyl-β-D-glucopyranosyluronate)morphin-3-yl terephthalate, and
- bis[6-*O*-(methyl 2,3,4-tri-*O*-acetyl-β-D-glucopyranosyluronate)-morphin-3-yl] terephthalate.

13. Compound of formula 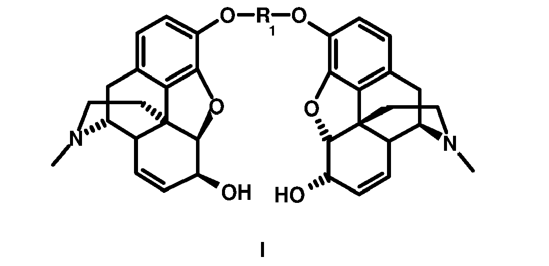 in which:
R₁ is as defined in Claim 1.

14. Compound according to Claim 13, chosen from the group formed by:
- dimorphin-3-yl terephtalate,
- dimorphin-3-yl isophthalate,
- dimorphin-3-yl phthalate,
- dimorphin-3-yl fumarate,
- dimorphin-3-yl benzene-1,2-disulfonate,
- dimorphin-3-yl benzene-1,3-disulfonate,
- dimorphin-3-yl thiophene-2,5-dicarboxylate,
- dimorphin-3-yl naphthalene-2,7-dicarboxylate,
- dimorphin-3-yl 4,4'-oxybenzoate,
- dimorphin-3-yl biphenyl-4,4'-dicarboxylate, and
- dimorphin-3-yl carbonate.
